# EUROPEAN PATENT APPLICATION

(11) **EP 1 157 708 A2**
(43) Date of publication of application: **28.11.2001**
(21) Application number: 01304569.5
(22) Date of filing: 24.05.2001
(51) Int. Cl.: A61L 31/16, A61L 17/00

(54) **Anti-microbial surgical devices**

(30) Priority: 25.05.2000 US 578955
(71) Applicant: ETHICON, INC., Somerville, NJ 08876 (US)
(72) Inventor: Fischer, Jerome A., Warren, NJ 07059 (US); Scalzo, Howard L., Kenilworth, NJ 07033 (US)
(74) Representative: Mercer, Christopher Paul

(57) **Abstract**

A surgical device is disclosed that contains an anti-microbial agent homogeneously dispersed there through in an amount effective to substantially prevent in-situ growth of bacteria on the device, which device may be prepared from an extruded, biocompatible filament and/or yarn containing the anti-microbial agent homogeneously dispersed there through in amounts effective to produce surgical devices capable of substantially preventing in-situ growth of bacteria on the surface thereof.

## Description

This invention relates to surgical devices containing an anti-microbial agent homogeneously dispersed there through.

### Background of the Invention

Surgical devices prepared from extruded filaments and/or yarn include mesh prostheses conventionally are used to repair hernias. Such mesh fabric prostheses also are used in other surgical procedures, including the repair of anatomical defects of the abdominal wall, diaphragm, and chest wall, correction of defects in the genitourinary system, and repair of traumatically damaged organs such as the spleen, liver or kidney. Mesh fabrics for use in connection with hernia repairs are disclosed in U.S. Patent Nos. 5,292,328, 4,769,038 and 2,671,444. Knitted and woven fabrics constructed from a variety of synthetic fibers and the use of the fabrics, in surgical repair are also discussed in U.S. Patent Nos. 3,054,406; 3,124,136; 4,193,137; 4,347,847; 4,452,245; 4,520,821; 4,633,873; 4,652,264; 4,655,221; 4,838,884;. 5,002,551; and European Patent Application No. 334,046.

Surgical devices prepared form extruded filaments and/or yarn also include mono- and multi-filament sutures. Such sutures are disclosed in, for example, United States Patents 4,557,264 and 4,911,165.

It is important during the healing process and subsequent thereto that the surgical devices placed within the body do not provide for the growth of bacteria on or immediately about the surgical device. Medical devices that utilize anti-microbial agents applied to their surfaces are known. For example, United States Patents 3,642,003 and 3,862,304 disclose sutures coated with germicidal ions. United States Patent 5,019,096 discloses devices, e.g. sutures, comprising coatings of antimicrobial agents. United States Patent 5,534,288 discloses substrates made from filaments, which substrates then are impregnated with an anti-microbial agent. The impregnating agent is said to flow into the interstices between the filaments from which the substrate is formed. It would be advantageous if medical devices, e.g. sutures, ligatures and mesh, prepared from filaments and/or yarn could be made to be anti-microbial without coating and/or impregnation processes such as those discussed above.

According to the present invention, medical devices are prepared having anti-microbial agents homogeneously dispersed there through. The devices are substantially resistant to growth of bacteria thereon without the need for additional surface treatment of the device, such as coating or impregnation.

### Summary of the Invention

The present invention is directed to a surgical device comprising an anti-microbial agent homogeneously dispersed there through in an amount effective to substantially prevent in-situ growth of bacteria on the device, which device may be prepared from a biocompatible, extruded filament and/or yarn comprising the anti-microbial agent homogeneously dispersed there through in amounts effective to produce surgical devices capable of substantially preventing in-situ growth of bacteria on the surface thereof.

### Detailed Description of the Invention

The surgical device of the present invention preferably is fabricated from a filament or yarn that is biocompatible and that comprises an anti-microbial agent homogeneously dispersed there through in an amount effective to produce surgical devices capable of substantially preventing in-situ growth of bacteria on the surface of such surgical devices. Homogeneously dispersed there through, as used herein, includes those filaments or yarns in which the anti-microbial agent is substantially, homogeneously dispersed there through, such that the anti-microbial properties of the filaments or yarns are effective to produce surgical devices capable of substantially preventing in-situ growth of bacteria on the device. Preferred are filaments and yarns that already have been accepted for use as a suture material or for mesh prostheses. Numerous biocompatible absorbable and non-absorbable filaments/yarns can be used to make the surgical devices described hereinafter.

Suitable non-absorbable materials for use in the present invention include, but are not limited to, polyamides (polyhexamethylene adipamide (nylon 66), polyhexamethylene sebacamide (nylon 610), polycapramide (nylon 6), polydodecanamide (nylon 12) and polyhexamethylene isophthalamide (nylon 61) copolymers and blends thereof), polyesters (e.g. polyethylene terephthalate, polybutyl terephthalate, copolymers and blends thereof), fluoropolymers (e.g. polytetrafluoroethylene and polyvinylidene fluoride) polyolefins (e.g. polypropylene including isotactic and syndiotactic polypropylene and blends thereof, as well as, blends composed predominately of isotactic or syndiotactic polypropylene blended with heterotactic polypropylene and/or polyethylene (such as is described in U.S. Patent 4,557,264 issued December 10, 1985, assigned to Ethicon, Inc., hereby incorporated by reference in its entirety) and combinations thereof.

Suitable absorbable materials for use as filaments and/or yarns include, but are not limited to, aliphatic polyesters which include but are not limited to homopolymers and copolymers of lactide (which includes L, D and meso lactide as well as L- and D- lactic acid), glycolide (including glycolic acid), ε-caprolactone, p-dioxanone (1,4-dioxan-2-one), trimethylene carbonate (1,3-dioxan-2-one), alkyl derivatives of trimethylene carbonate, δ-valerolactone, β-butyrolactone, γ-butyrolactone, ε-decalactone, hydroxybutyrate, hydroxyvalerate, 1,4-dioxepan-2-one (including its dimer 1,5,8,12-tetraoxacyclotetradecane-7,14-dione), 1,5-dioxepan-2-one, 6,6-dimethyl-1,4-dioxan-2-one and polymer blends thereof.

Filaments and/or yarns may be made from absorbable and non-absorbable materials described above in heterologous yarns or bicomponent yarns. Additionally, filaments with different materials used in the sheath and core also may be used for the inventive surgical meshes.

Surgical filaments such as sutures and ligatures that contain anti-microbial effective amounts of an anti-microbial agent are included within the present invention. Exemplary filaments are described in, for example, United States Patent 4,557,264, and United States Patent 4,911,165, assigned to Ethicon, Inc., the contents of which is hereby incorporated by reference in its entirety. As noted in United States Patent 4,911,165, polypropylene pellets are introduced into an extruder and the extruded filament then is drawn to predetermined draw ratios. According to the present invention, the anti-microbial agent first is homogeneously dispersed throughout the polymer resin in powder form. The resin may be introduced directly into the extruder in powder form, or the resin containing the anti-microbial agent may be formed into pellets, which then are introduced into the extruder. The extruded filament or yarn, containing anti-microbial amounts of the anti-microbial agent, may be used then to prepare medical devices according to the present invention.

In view of the process conditions to which the anti-microbial agent may be exposed during preparation of the polymer resin, extrusion of the filament and/or yarn and subsequent production of the medical device itself, the anti-microbial agent must be one capable of being exposed to such conditions without undergoing chemical degradation or modification, or significant loss of anti-microbial properties. The agents also must be homogeneously dispersible in the polymer resin used to prepare the extruded filaments and/or yarns.

Anti-microbial agents utilized in the present invention may be selected from the group consisting of phenol derivatives, such as chlorophene, dichloroxylenol, hexachloraphane, diphenyl derivatives, halogenated hydroxy diphenyl derivatives, such as diphenyl ethers, e.g. 2,4,4'-trichloro-2'-hydroxy diphenyl ether, diacetylaminoazotoluene and triclocaban. Preferred anti-microbial agents comprise diphenyl ethers, in particular 2,4,4'-trichloro-2'-hydroxy diphenyl ether.

Filaments and/or yarns used to prepare medical devices of the present invention are prepared by extrusion of a polymer resin. During manufacture of the filament and/or device, some of the anti-microbial agent typically is lost due to process steps and conditions. The amount of anti-microbial agent used in preparing the polymer resin is determined based on the amount of agent actually required present in the medical device to substantially prevent in-situ growth of bacterial on the surface of the device, taking into account the potential loss of agent due to process steps and conditions. The resin comprises an anti-microbial agent substantially homogeneously dispersed there through in amounts effective to produce extruded filaments and/or yarns that, in turn, are useful in preparing medical devices capable of substantially preventing in-situ growth of bacteria on the surface thereof.

The compounding of the antimicrobial agent into the polymer is performed in several steps. The polymer resin and antimicrobial agent are fed through a compounding device, such as a twin-screw extruder. Feeding can be accomplished by force, gravity or starve feed systems. The extruder heats and mixes the materials until they are uniformly blended. A twin-screw extruder produces good mixing by forcing the melt back and forth from one screw to the other. thus breaking up flow patterns. The melted compound exits the extruder in strands that are quenched (cooled/crystallized) by water or air and cut into pellets. Those pellets can be then fed into a second extruder where the actual fiber will be manufactured.

The agent is homogeneously dispersed in the resin in a minimum amount effective to prepare extruded filaments and/or yarns that, in turn, comprise the anti-microbial agent homogeneously dispersed there through in amounts effective to produce medical devices capable of substantially preventing in-situ growth of bacteria on the surface thereof. Accordingly, the polymer resin must comprise a minimum amount of the anti-microbial agent such that, upon manufacture of the medical device, the medical device will be effective to substantially prevent in-situ growth of bacteria on its surface. While such devices may have trace amounts of bacteria present thereon, the levels of bacteria generated in-situ will be sufficiently low so as not to lead to infection or require additional anti-microbial agents applied to the surface of the device in order to avoid infection, e.g. surface coatings containing anti-microbial agents. The maximum amount of the anti-microbial agent that can be used will be limited by the desired properties of the filament/yarn and surgical devices. For example, excessive amounts of the agent may over-plasticize or otherwise detrimentally affect the properties of the device. Accordingly, the polymer resin may comprise from about 0.5 to about 10 weight percent ofthe anti-microbial agent, based on total weight of the polymer resin and agent. Preferably, the polymer resin will comprise from about 0.5 to about 5 weight percent ofthe anti-microbial agent. Once having the benefit of the present invention, one skilled in the art will readily ascertain the effective minimum and maximum amounts of the anti-microbial agent.

Preferably, the filament or yarn used to prepare the devices of the present invention, as well as the surgical device of the present invention, will comprise from about 0.5 to about 5 weight percent of the anti-microbial agent, more preferably from about 0.75 to about 2.5 weight percent of the anti-microbial agent, based on total weight of the filament, yarn or device.

A surgical mesh of the present invention may be fabricated from a 6 mil diameter monofilament polypropylene yarn comprising anti-microbial effective amounts of an anti-microbial agent by employing known and conventional warp knitting apparatus and techniques, such as the tricot and Raschel knitting machines and procedures described in "Warp Knitting Production" by Dr. S. Raz, Melliand Textilberichte GmbH, Rohrbacher Str. 76, D-6900 Heidelberg, Germany (1987). As is well known in the art of warp knitting, the number of courses and wales per inch in a knitted material is affected by a number of machine operating variables such as the rate at which the fabric is drawn away from the needles, the number of needles per inch, the amount of tension applied to the warp yarns and other variables after the fabric leaves the machine, e.g., the heat setting conditions.

In one embodiment of the present invention, polypropylene monofilament yarn described above is warp knitted, preferably tricot knitted on a 3 bar set-up, in accordance with the parameters set forth in Table A below:

**TABLE A**

| **Courses per Inch** | **Wales per Inch** | **Back Bar** | **Middle Bar** | **Front Bar** |
|---|---|---|---|---|
| 6-10 | 7-11 | 1-0 | 1-0 | 2-3 |
| | | 1-2 | 2-3 | 2-1 |
| | | 1-0 | 2-3 | 2-3 |
| | | 1-2 | 1-0 | 2-1 |
| | | 1-0 | 1-0 | 2-3 |
| | | 2-3 | 2-3 | 1-0 |
| | | 2-1 | 2-3 | 1-2 |
| | | 2-3 | 1-0 | 1-0 |

Guide bar set-up threaded xoxo on all three bars.

Following knitting, the mesh is cleaned or scoured, and thereafter annealed to stabilize the fabric. For the latter operation, the mesh can be secured to a tenter frame which maintains the mesh at a predetermined width, the frame then being passed through an elongated heating zone at a temperature of from about 100°C to about 160°C, preferably at a temperature of from about 120°C to about 150°C, at a rate providing a dwell time of from about 0.5 to about 60 minutes and preferably from about 1.5 to about 25 minutes. Following heat setting, the mesh is cut to size, packaged and sterilized.

The mesh can be cut to any desired configuration, e.g., a square or rectangular shape of appropriate dimensions. An ultrasonic slitter may be employed to cut the mesh, various types of which are commercially available. Unlike the result one obtains when cutting with a blade, i.e., frayed yarn ends, or when the yarn ends are heat-sealed, i.e., bead-like formations, cutting the mesh to size with an ultrasonic cutter avoids both frayed and beaded ends.

The polypropylene monofilament knitted mesh fabricated as described above exhibits good pliability. Depending on the yarn used to form the mesh, a mesh formed in accordance with Table A above preferably has a flexibility of about 700-850 mg-cm. In addition, depending on the yarn used to form the mesh, a mesh formed in accordance with Table A above preferably has a burst strength of about 200-250 pounds per square inch and, when the 6 mil diameter monofilament yarn described above is used, the mesh has a mean burst strength of 230 pounds per square inch. Actual burst strength varies between about 220 to about 240 pounds per square inch, depending on the sample. Finally, depending on the yarn used to form the mesh, a mesh formed in accordance with Table A above preferably has a pore size percentage greater than 50%, and more preferably of from about 55% to about 70%. When the 6 mil diameter monofilament yarn described above is used, the mesh has a pore size of about 65%. The polypropylene monofilament knitted mesh fabricated as described above preferably possesses a thickness of from about 22 to about 26 mils, depending on the particular yarn used. When the 6 mil diameter monofilament yarn described above is used, the mesh has a mean thickness of 24 mils. Actual thickness varies between about 23 to about 25 mils, depending on the sample. The pore size percentage is the percentage of the area in the plane of the mesh not blocked by the fibers of the knitted mesh.

### Examples

The following examples illustrate the anti-microbial characteristics of a polypropylene surgical mesh formed from a monofilament yarn comprising no anti-microbial agent, i.e. a non-treated mesh, compared to a polypropylene mesh formed from a monofilament yarn comprising about 2% by weight of 2,4,4'-trichloro-2'-hydroxydiphenol, i.e. a treated mesh.

### Example 1

One-inch square pieces of a non-treated surgical mesh and a treated surgical mesh were added to sterile Tryptic Soy Broth (TSB) in 40 mL test tubes with Morton closures. The tubes were inoculated with 1 mL of a 2.8 x 10² cfu/mL culture of *Staphylococcus aureus* ATCC 6538. Control tubes of TSB with no inoculum and no mesh and TSB with inoculum and no mesh were also prepared. The tubes were incubated at 35°C. Separate sets of tubes were prepared for each of the following sample times: 0, 1, 4, and 6 hours.

At each sample time, duplicate 1 mL aliquots were removed from each tube of the sample time set. The aliquots were diluted and pour plated with 15-20 mL of TSB and incubated at 35°C.

After the tubes in each sample set had been pour plated and measured for turbidity, the mesh pieces were removed and placed into 200 mL of 0.85% saline with 0.01% Tween 80 and shaken for 60 minutes on low speed on an Eberbach shaker. After shaking, 100 mL of the wash solutions from each sample were pour plated in 25 mL increments with TSB. The data for treated and non-treated samples listed in Table 2 was calculated using a multiplication factor of 2 since only one-half of each 200 mL wash solution was pour plated and counted.

### Example 2

One inch square pieces of a non-treated mesh and a treated mesh were added to sterile TSB in 40 mL test tubes with Morton closures. The tubes were inoculated with 1 mL of Staphylococcus aureus ATCC 33591 (methicillin resistant) at approximately 4.5 x 10⁴ cfu/mL or 1 mL of Staphylococcus epidermidis ATCC 51625(methicillin resistant) at approximately 1.8 x 10² cfu/mL. Control tubes of TSB with no inoculum and no mesh and TSB with inoculum and no mesh were also prepared. The tubes were incubated at 35°C. Separate sets of tubes were prepared for each of the following sample times: 0, 1, 6, 24 and 48 hours.

At each sample time, duplicate 1 mL aliquots were removed from each tube of the sample time set. The aliquots were diluted and pour plated with 15-20 mL of TSB and incubated at 35°C for 48 hours.

After the tubes in each sample set had been poured, the mesh pieces were removed and placed into 200 mL of 0.85% saline with 0.01% Tween 80 and shaken for 60 minutes on low speed on an Eberbach shaker. After shaking, 100 mL of the wash solutions from each sample were pour plated in 25 mL increments with TSB. The data for treated and non-treated samples listed in Table 4 was calculated using a multiplication factor of 2, since only one-half of each 200 mL wash solution was pour plated and counted.

Tables 1 and 2 list the average cfu/mL recovered for the two pour plate recovery tests using of *Staphylococcus aureus* ATCC 6538 (Example 1). Tables 3 and 4 include 24 and 48 hour data using Staphylococcus aureus ATCC 33591 (methicillin resistant) or 1 mL of Staphylococcus epidermidis ATCC 51625(methicillin resistant) (Example 2).

The viable count data listed below indicate that treated mesh inhibited bacterial growth more than non-treated mesh during the course of the experiment. Non-treated mesh showed no measurable inhibition of growth of the test organisms.

**Table 1.**

| **Broth Recovery (average of 2 plates)** | | | |
|---|---|---|---|
| **Time (hours)** | **Treated mesh cfu** | **Non-treated mesh cfu** | **Control cfu** |
| 0 | 0 | 0 | 0 |
| 1 | 8.5 | 6.5 | 7.5 |
| 4 | 1 | 45.5 | 47.5 |
| 6 | 0 | TNTC | TNTC |
| TNTC = Too Numerous To Count | | | |

**Table 2.**

| **Wash and Recovery for attached organisms (average of 4 plates)** | | |
|---|---|---|
| **Time (hours)** | **treated (cfu/mL)** | **non-treated (cfu/mL)** |
| 0 | 0 | 0 |
| 1 | 0.5 | 0.5 |
| 4 | 1.0 | 15 |
| 6 | 0.02 | 425.5 |

**Table 3.**

| **Broth Recovery (average of 2 plates)** | | | |
|---|---|---|---|
| **Time (hours)** | **treated cfu** | **Non-treated cfu** | **Control cfu** |
| S. aureus | | | |
| **0** | **58** | **48** | **48** |
| 1 | 126 | 242 | 65 |
| 6 | 75 | 245 | 442 |
| 24 | 1.1x10³ | 2.0x10⁸ | 4.2x10⁸ |
| 48 | 0 | 5.0x10⁸ | 5.3x10⁸ |

| S. epidermidis | | | |
|---|---|---|---|
| 0 | 0.5 | 0 | 0 |
| 1 | 1.5 | 0.5 | 1 |
| 6 | 0 | 83 | 83 |
| 24 | 0 | 6.7x10⁷ | 5.7x10⁷ |
| 48 | 0 | 2.3 x10⁸ | 4.1 x10⁸ |

**Table 4.**

| **Wash and Recovery for attached organisms (average of 4 plates)** | | |
|---|---|---|
| **Time (hours)** | **treated (cfu/mL)** | **non-treated (cfu/mL)** |
| S. aureus | | |
| 0 | 0 | 2.5 |
| 1 | 4.7 | 7 |
| 6 | 1.7 | 174 |
| 24 | 7.8 | TNTC |
| 48 | 0 | TNTC |

| S. epidermidis | | |
|---|---|---|
| 0 | 0 | 0 |
| 1 | 0 | 0 |
| 6 | 0 | 1.3 |
| 24 | 0 | TNTC |
| 48 | 0 | TNTC |
| TNTC = Too Numerous To Count | | |

## Claims

1. A surgical device, comprising:
an anti-microbial agent homogeneously dispersed therethrough in an amount effective to substantially prevent in-situ growth of bacteria on a surface of the device.

2. The device of claim 1 comprising an extruded, biocompatible filament and/or yarn comprising the anti-microbial agent homogeneously dispersed therethrough in an amount effective to substantially prevent in-situ growth of bacteria on the surface thereof.

3. The device of claim 2 which is a suture, a ligature or a surgical mesh.

4. The device of claim 3, which is a surgical mesh wherein the yarn is a non-absorbable yarn made from cotton, linen, silk, a polyamide, a polyester, a fluoropolymer or a polyolefin.

5. The surgical mesh of claim 4, wherein the yarn is made from polypropylene fiber.

6. The device of claim3, which is a surgical mesh wherein the yarn is an absorbable yarn made from a homopolymer or copolymer of lactide, glycolide, ε-caprolactone, p-dioxanone (1,4-dioxan-2-one), trimethylene carbonate (1,3-dioxan-2-one), an alkyl derivative of trimethylene carbonate, δ-valerolactone, β-butyrolactone, γ-butyrolactone, ε-decalactone, hydroxybutyrate, hydroxyvalerate, 1,4-dioxepan-2-one and its dimer 1,5,8,12-tetraoxacyclotetradecane-7,14-dione, 1,5-dioxepan-2-one or 6,6-dimethyl-1,4-dioxan-2-one.

7. The device of any one of claims 1 to 6 comprising from 0.5 to 5 percent by weight, preferably from 0.75 to 2.5 percent by weight, of the anti-microbial agent.

8. The device of any one of claims 1 to 7 wherein the anti-microbial agent is a phenol derivative, a diphenyl derivative, diacetylamino-azotoluene, triclocaban or a mixture thereof.

9. The device of claim 8 wherein the anti-microbial agent is chlorophene, dichloroxylenol, hexachloraphane, 2,4,4'-trichloro-2'-hydroxy diphenyl ether, diacetylamino-azotoluene or triclocaban.

10. The device of claim 9 wherein the anti-microbial agent is 2,4,4'-trichloro-2'-hydroxy diphenyl ether.
